# EUROPEAN PATENT APPLICATION

(11) **EP 4 545 089 A1**
(43) Date of publication of application: **30.04.2025**
(21) Application number: 23206427.9
(22) Date of filing: 27.10.2023
(51) Int. Cl.: A61K 38/28, A61K 38/50, A61P 35/00

(54) **COMBINED ENZYMATIC-METABOLIC CANCER THERAPY**

(71) Applicant: Kyon Biotech AG, 8005 Zürich (CH)
(72) Inventor: TEPIC, Slobodan, 8057 Zurich (CH); CVETKOVIC, Goran, 8645 Jona (CH); CVETKOVIC, Olivera, 8645 Jona (CH)
(74) Representative: Weickmann & Weickmann PartmbB

(57) **Abstract**

The present invention relates to a treatment of cancer combining enzymatic with metabolic means of selective attack on cancer cells.

## Description

The present invention relates to a treatment of cancer combining enzymatic with metabolic means of selective attack on cancer cells.

### Background

Depletion of L-arginine (in the following arginine) has been shown of utility in treating some cancers such as hepatocellular carcinoma and melanoma, and based on *in vitro* work, probably many others. The use of arginine-depleting enzymes such as arginase in cancer therapy has e.g., been described by Shen et al. (Cell Death & Disease 8 (2017), e2720), Zou et al. (Biomedicine & Pharmacotherapy 118 (2019), 109210), Al-Koussa et al. (Cancer Cell International 20 (2020) Article number 150) and Zhang et al. (Cancer Letters 502 (2012), 58-70), the contents of which are herein incorporated by reference.

The use of arginine-depleting enzymes is necessary, but our own research has shown it is not sufficient to cause and maintain systemic, deep depletion of arginine needed to cause rapid, selective killing of cancer cells.

The use of an insulin/glucose clamp in parallel with the enzymatic degradation of arginine makes the task of deep arginine depletion much more manageable. Insulin is a growth factor and thus promotes protein synthesis and inhibits protein breakdown. This is of crucial importance when the task is removing an amino acid from circulation, particularly removing arginine, which is a semi-essential amino acid under tight homeostatic control.

An increase of vascular permeability by insulin also helps in getting therapeutic enzymes into interstitial fluid space, closer to where most cancerous cells reside. Finally, insulin may also play a role in transporting arginine-degrading enzymes into cancerous cells by stimulating endocytosis.

However, as the inventors' research has shown, even with the use of an insulin/glucose clamp, free arginine levels could only be reduced to about 5 to 10 µM, i.e., to about 5 to 10% of the normal plasma concentration of about 100 µM. At these free arginine levels, cells, cancerous and healthy, will not proliferate but still can survive for prolonged periods. The target for arginine concentration to result in rapid killing of cancer cells is 1 µM or less. Healthy cells can survive such low levels of arginine much longer than cancer cells (weeks vs days) which accounts for the selectivity of this intervention.

The main obstacle to achieving this level of arginine depletion is the conversion of citrulline into arginine by the kidneys. Citrulline is produced by intestinal lining cells from ornithine which in turn is produced from non-essential amino acids glutamine and proline.

The inventors have attempted inhibition of citrulline production in the intestines but none of the many approaches tested in experimental dogs have resulted in the satisfactory reduction of plasma citrulline.

The resolution to the problem of citrulline as a precursor of arginine was found in the use of argininosuccinate synthase (ASS) which converts citrulline and aspartate to argininosuccinate. ASS is delivered to the vascular system where it interrupts the transport of citrulline from the intestines to the kidneys. In the urea cycle of the liver, argininosuccinate is converted to arginine by argininosuccinate lyase (ASL) but this does not happen in the vascular system. Accordingly, WO 2023/066910, the content of which is herein incorporated by reference, describes a medicament comprising an arginine decomposing enzyme such as arginase (ARG) and a citrulline converting enzyme such as argininosuccinate synthase (ASS) for the treatment of cancer.

Certain blood cancers, e.g., acute lymphoblastic leukemia (ALL) or non-Hodgkin lymphoma, are highly susceptible to depletion of asparagine, a non-essential amino acid. The use of L-asparaginase (ASNase) in treating these blood cancers is a highly successful precedent for all other amino-acid depletion schemes.

Unlike arginine, asparagine is easily depleted from circulation for the apparent lack of any systemic mechanisms of homeostasis. L-asparaginase also degrades glutamine, another non-essential amino acid. Glutamine is used as a precursor for many biosynthetic pathways and is found in abundance in all cells and in all extracellular fluids.

WO 2020/245041, the content of which is herein incorporated by reference, discloses delivery of asparaginase (ASNase) in a dissociated form, particularly in the form of monomeric units.

WO 2022/034218, the content of which is herein incorporated by reference, discloses that the efficacy of biologics may be increased by assisting the extravasation of these compounds by co-administering a biological therapeutic molecule together with insulin and glucose.

It was an object of the present invention to provide means for improving the depletion of free arginine in the blood of a subject, particularly of a cancer patient. More particularly, it was an object of that invention to overcome disadvantages associated with previous treatment schedules involving amino acid depletion with, e.g., administration of PEGylated arginase (ARG) or PEGylated arginine deiminase (ADI), which are currently investigated in over 30 clinical trials for cancer treatment.

### Summary of the invention

The present inventors have found that the combined catalytic activity of these three enzymes ARG, ASS, and ASNase together with insulin but under conditions of glucose depletion leads to the systemic reduction of arginine, citrulline, asparagine, and glutamine. Most cancer cells are rapidly - in a matter of one or a few days - eliminated while the healthy cells survive with minimal losses. Healthy, normally proliferating cells such as those of the intestinal lining or bone marrow, exit the cycle but can resume proliferation once these amino acids are brought back to normal physiological levels.

A first aspect of the present invention relates to an amino acid-degrading enzyme for use in medicine, wherein a subject in need thereof is subjected to a treatment with at least one amino acid-degrading enzyme and a blood glucose-lowering agent under conditions of glucose depletion.

A further aspect of the present invention relates to a method for the treatment of cancer wherein a subject in need thereof is subjected to a treatment with at least one amino acid-degrading enzyme and a blood glucose-lowering agent under conditions of glucose depletion.

In certain embodiments, the amino acid-degrading enzyme is selected from:
- an asparaginase, particularly in a dissociated form, particularly in the form of monomers;
- an arginase;
- an argininosuccinate synthase;
- a glutaminase;
- or any combination thereof.

The present invention combines two powerful attacks on cancer cells - systemic depletion of amino acids for protein synthesis and depletion of glucose. In particular, the amino acids targeted are arginine and glutamine, together with citrulline and asparagine. The preferred enzyme for the degradation of arginine is arginase (ARG). The preferred enzyme for the degradation of glutamine is asparaginase (ASNase), which converts asparagine to aspartic acid but also glutamine to glutamic acid.

Our experimental clinical studies with ASNase for canine lymphoma have shown variable effectiveness of ASNase in lowering systemic levels of glutamine. It is therefore an option to use glutaminase as the fourth enzyme. Our recent in vitro research gives a strong rationale for combining these enzymes. Exposed to arginase, cancer cells have shown decreased intracellular concentrations of all amino acids but of asparagine and glutamine that have increased. While it is not clear why, it seems reasonable to assume that removing these two amino acids with asparaginase and glutaminase should have a synergetic effect with arginase.

Further, glucose is systemically lowered by the use of a glucose-lowering agent such as insulin.

Furthermore, the lack of glucose may be supplemented by administration of a ketone body compound.

### Description of preferred embodiments

According to the present invention, a subject in need thereof is subjected to a treatment with at least one amino acid-degrading enzyme and a blood glucose-lowering agent under conditions of glucose depletion. The term "amino acid-degrading enzyme" relates to an enzyme which is capable of reducing the amount of an amino acid under physiological conditions. In certain embodiments, the present invention comprises a treatment involving reducing the amount of the amino acids arginine, citrulline, asparagine and glutamine in order to provide conditions which are detrimental for the survival of cancer cells within the body of a subject to be treated.

In certain embodiments, the present invention relates to the depletion of arginine by enzymatic means for use in medicine, particularly for the treatment of cancer, in combination with the concurrent depletion of glucose and glutamine.

The inventors have found that systemic delivery of an arginine-decomposing enzyme such as an arginase (ARG) and a citrulline-converting enzyme such as argininosuccinate synthase (ASS) in partially purified liver extracts prepared by low-temperature protocols has led to a reduction of free plasma arginine to below detection levels. This was the basis of co-owned application WO 2023/066910, supra, describing a medicament comprising an arginine-decomposing enzyme such as an ARG and a citrulline-converting enzyme such as an ASS for use in a deep depletion of arginine levels in circulating blood.

Enzymatic decomposition of arginine by e.g., an arginase (ARG) was found to be necessary but not sufficient for a deep, systemic depletion of arginine. A concurrent removal of citrulline on its passage from intestines, the main organ for citrulline synthesis, to kidneys where citrulline is converted to arginine, by a citrulline-converting enzyme, e.g., argininosuccinate synthase (ASS) enables systemic reduction of free arginine to a concentration below detection by standard amino acids analysis. This leads to the rapid killing of cancerous cells of many if not all cancer types. Healthy dividing cells respond by exiting into G₀ phase and very few suffer terminal damage.

In certain embodiments, the arginine-decomposing enzyme is an arginase (ARG), i.e., an enzyme, which catalyzes the hydrolysis of L-arginine to L-ornithine and urea (EC. 3.5.3.1). Typically, the arginase is a mammalian arginase, e.g., a human arginase including any enzymatically active fragment and derivative thereof. In particular embodiments, the arginase is selected from human arginase-1 (liver arginase or ARG1) (UniProt-P05089) or human arginase 2 (kidney arginase or ARG2) (UniProt-P78540) including any enzymatically active fragment and derivative thereof. In even more particular embodiments, the arginase is human ARG1.

Human ARG1 is a 322 amino acid long polypeptide with a predicted molecular weight of 34,735 Da. It may be present in a monomeric or multimeric, e.g., trimeric form. Preferably, it is present in a monomeric form, which is the native form in the liver (personal communication from Prof. M. Ikemoto, U. of Kyoto, who was the first to clone ARG1). The preparation of recombinant ARG1 in *E*. *coli* is described by Ikemoto et al. in: Expression of human liver arginase in Escherichia coli. Purification and properties of the product. Biochem J. 1990 Sep 15;270(3):697-703. doi: 10.1042/bj2700697. PMID: 2241902; PMCID: PMC1131788; the content of which is herein incorporated by reference.

In certain embodiments, the arginase is a recombinant human ARG1 including any enzymatically active fragment and derivative thereof. In particular embodiments, the arginase is an unPEGylated polypeptide, i.e., it is not conjugated to a polyethylene glycol moiety.

A modification of human ARG1 to replace manganese with cobalt and to shift the optimum pH to that of plasma is also particularly suitable according to the present invention (Stone EM, Glazer ES, Chantranupong L, et al. Replacing Mn(2+) with Co(2+) in human arginase enhances cytotoxicity toward l-arginine auxotrophic cancer cell lines [published correction appears in ACS Chem Biol. 2010 Aug 20;5(8):797]. ACS Chem Biol. 2010;5(3):333-342. doi:10.1021/cb900267j), the content of which is herein incorporated by reference.

In certain embodiments, arginase, e.g., human ARG1 is administered as an arginase-insulin fusion protein as described in co-owned application WO 2023/041758, the content of which is herein incorporated by reference.

In another particular embodiment, the arginine-decomposing enzyme is an arginine deiminase, e.g., an arginine deiminase of 46 kDa (UniProt-A0A0C6G6L6_MYCAR).

In another particular embodiment, the arginine-decomposing enzyme is an arginine decarboxylase, e.g., a biosynthetic arginine decarboxylase of 74 kDa (UniProt-Q8FE34 SPEA_ECOL6) or a biodegradative arginine decarboxylase of 80 kDa (UniProt-A0A376VTJ3_ECOLX).

Arginase may be supplied externally, but an alternative is its endogenous release from the liver which would, due to low glucose, undergo a degree of necrosis and/or apoptosis.

In certain embodiments, the citrulline-converting enzyme is an argininosuccinate synthase (ASS), i.e., an enzyme, which catalyzes the conversion of L-aspartate and L-citrulline to L-argininosuccinate (EC 6.3.4.5). Typically, the ASS is a mammalian ASS, e.g., a human ASS including any enzymatically active fragment and derivative thereof. In particular embodiments, the ASS is human argininosuccinate synthase (ASS1) (UniProt-P00966) including any enzymatically active fragment and derivative thereof.

Human ASS1 is a 412 amino acids long polypeptide with a predicted molecular weight of 46,530 Da. It may be present in a monomeric or multimeric, e.g., tetrameric form. Preferably, it is present in a tetrameric form, which is its native active form in the human liver.

In certain embodiments, the ASS is a recombinant human ASS1 including any enzymatically active fragment and derivative thereof. In certain embodiments, the ASS is a PEGylated polypeptide. In other embodiments, the ASS is an unPEGylated polypeptide.

In certain embodiments, ASS is needed to bring arginine to a very low concentration. It also is released by the liver due to the gradual, partial loss of hepatocytes deprived of glucose. ASS may be supplied externally, but an alternative is its endogenous release from the liver.

In certain embodiments, ARG and ASS are present as at least partially purified mammalian liver extract. A preferred method for obtaining a suitable liver extract is described in the Examples of the co-owned application WO 2023/066910, supra. In certain embodiments, ARG and ASS are recombinant polypeptides.

In certain embodiments, both ARG and ASS are supplied by external administration, e.g., infusion.

In certain embodiments, an external administration of ARG and/or ASS is not required.

Another aspect of research by the inventors was the modification of the delivery of L-asparaginase (ASNase). ASNase can be purified from certain strains of *E*. *coli* or can alternatively be produced by recombinant technology from genetically modified strains of *E*. *coli.* ASNase is a commonly used drug to treat blood cancers, e.g., leukemias and lymphomas.

In a particular embodiment, the ASNase its in its tetrameric (active) form of 140 kDa, or in its monomeric form of 35 kDa.

In an even more particular embodiment, the ASNase is in a dissociated form, particularly in the form of a monomer. The reversible dissociation of ASNase into its monomers by a high concentration of a chaotropic agent such as urea as a functional excipient facilitates the extravasation of monomers which are then reconstituted in the interstitial fluid in the surrounding of most of the cancer cells of any type of cancer, including blood cancers, as disclosed in the co-owned application WO 2020/245051, supra.

The asparaginase may be administered as an aqueous preparation comprising urea, particularly in a concentration between about 3 mol/l to about 8 mol/l, more particularly in a concentration of about 4 mol/l to about 6 mol/l, e.g., about 5 mol/l.

Further, the present invention comprises administration of a blood glucose-lowering agent, e.g., an insulin or an insulinotropic peptide, particularly a fast-acting insulin. Insulin is a growth factor that also is a glucose-lowering hormone.

In certain embodiments, the insulin is administered as an arginase-insulin fusion protein as described as described in co-owned application WO 2023/041758, supra.

The treatment according to the present invention is carried out under conditions of glucose depletion. Glucose depletion is understood as a blood glucose level of lower than the normal glucose level of the subject to be treated. In certain embodiments, conditions of glucose depletion involve adjusting and maintaining a blood glucose level of about 2 mM or less, e.g., about 0.8 mM to about preferably to as low as about 1 mM. Glucose depletion may be achieved by administration of a blood glucose-lowering agent as described herein.

In certain embodiments, conditions of glucose depletion are maintained throughout a treatment cycle, e.g., during the administration of the amino acid-degrading enzyme. In certain embodiments, the conditions of glucose depletion are maintained for at least 12 h, at least 24 or at least 48 h.

In certain embodiments, the blood glucose-lowering agent is administered without concomitant administration of glucose or a glucose precursor, e.g., a glucose-containing oligo or polysaccharide.

Furthermore, the present invention may comprise the administration of a ketone body compound.

The brain can use ketone bodies and its glial cells are also capable of producing ketone. During the treatment, most of the energy needs of the healthy cells are fulfilled by the administration, e.g., infusion of ketone bodies, preferably in the form of 3-hydroxybutyric acid, or a physiologically acceptable 3-hydroxybutyrate salt, e.g., an alkaline metal, alkaline earth metal or amino acid salt. In certain embodiments, the salt is selected from sodium, potassium, calcium, and magnesium 3-hydroxybutyrate or a combination thereof. The use of a balanced mixture of those salts may avoid the overloading of sodium while providing the needed amount of butyrate. Administering an amino acid salt, e.g., lysine 3-hydroxybutyrate, is of special interest since lysine is an antagonist of arginine. An alternative to infusion is exogenous oral delivery. Residual systemic glucose usually provides the minimum needed to support the brain, liver, and erythrocytes.

In certain embodiments, glutaminase (GLS) is administered, particularly in combination with ASNase and optionally ARG and/or ASS. GLS can be added should ASNase fail to sufficiently lower glutamine. In dogs treated with asparaginase, glutamine concentration could be lowered to between the detection limit (<1 micromolar) and 100 micromolar, from the normal level of close to 1 mM. An effective reduction calls for glutamine of < 50 micromolar, preferably of < 10 micromolar.

In certain embodiments, the GLS is a kidney-type GLS, e.g., human kidney (GLSK) with 669 amino acids and a molecular weight of 73,461 Da or any processed form thereof such as Isoform 3 of 68 kDa, or a liver-type GLS, e.g., human liver GLS (LGA)as described by Katt et al (Future Med Chem 2017, 9(2), 223-243, and 9(5), 527), the content of which is herein incorporated by reference In certain embodiments, the GLS is a recombinant polypeptide.

A particular aspect of the invention relates to a medicament comprising the following active agents:
(i) an asparagine-decomposing enzyme such as an ASNase;.
(ii) a blood glucose-lowering agent, e.g., an insulin;
(iii) a ketone body compound, e.g., a physiologically acceptable 3-hydroxybutyrate salt;
(iv) an arginine-decomposing enzyme such as an ARG;
(v) a citrulline-converting enzyme such as an ASS, and
(vi) optionally a glutamine-decomposing enzyme such as a GLS.

In certain embodiments, ARG and/or ASS need not be added externally but are generated endogenously as described above.

The medicament of the invention is suitable for use in human medicine and veterinary medicine, e.g., for the treatment of dogs. While the use of the human versions of ARG and ASS is acceptable in treating dogs, canine versions of ARG and ASS are preferred. Both have been produced as recombinant proteins expressly for use in dogs by the present inventors.

Administration of the medicament in a therapeutically active dose will lead to a deep depletion of arginine levels in the blood. In certain embodiments, the arginine levels are about 20 µM or less for a time of at least 12 h or even for at least 72 h, preferably the arginine levels are about 10 µM or less for a time of at least 12 h or even for at least 72 h, more preferably the arginine levels are about 5 µM or less for a time of at least 12 h or even for at least 72 h, and most preferably the arginine levels are about 1 µM or less for a time of at least 12 h or even for at least 72 h as measured in the venous blood plasma. Citrulline concentration in the human plasma of healthy individuals is 30 to 50 µM. Observations from human patients with advanced hepatocellular carcinoma that the inventors participated in treating with arterial occlusion of the liver suggest that lowering citrulline level two to five-fold is sufficient to lower arginine level to the target of less than 1 µM. In dogs normal citrulline concentration is about two times higher than in humans but the same reduction of two to five times was correlated with successful arginine depletion.

In certain embodiments, the medicament of the present invention comprises five active agents. Three are amino acid degrading enzymes, one or two of which (ARG and ASS) may be added externally or released endogenously from the liver deprived of the minimum required circulating glucose. L-asparaginase (ASNase) is the first active agent and in all cases it is supplied externally by infusion. The second active agent is a blood glucose-lowering agent such as insulin. The third is a ketone body compound, preferably 3-hydroxybutyrate or a physiologically acceptable salt thereof such as sodium 3-hydroxybutyrate. The fourth and the fifth active agents are ARG and ASS, respectively. The active agents are administered together with pharmaceutically acceptable excipients, e.g., selected from buffers, salts, and/or stabilizers

The medicament may be a pharmaceutical preparation comprising each of the active agents separately. Alternatively, the medicament may be a combination of three separate pharmaceutical preparations, one comprising ASNase dissolved in urea, the other comprising ARG and ASS, and the third comprising insulin added to a solution of a ketone body compound such as 3-hydroxybutyrate.

The pharmaceutical preparation may be a liquid pharmaceutical preparation comprising the active agent(s) in dissolved or suspended form ready for use.

Alternatively, the pharmaceutical preparation may be a solid pharmaceutical preparation comprising the active agent(s) in lyophilized or freeze-dried form for reconstitution with a suitable solvent, e.g., an aqueous solvent.

The medicament is administered in therapeutically effective doses of the active agents.

Asparaginase (ASNase) in conventional clinical use for blood cancers in people and in dogs is typically given at a dose of 400 IU/kg, 3 times a week for about 3 months. Our own research has demonstrated that this is greatly inadequate. To reach the levels of the enzyme in the interstitial fluid (ISF) needed to exert a relevant effect on the cancer cells based on what is known from the dose-response studies *in vitro* we have been using in experimental and clinical studies in animals 3000 IU/kg. To maintain the enzymatic activity in the ISF, a daily dose of at least about 1000 IU/kg, e.g., about 3000 IU/kg, may be delivered to the subject in need thereof, e.g., a huma or dog. The daily dose is preferably delivered by a continuous infusion, e.g., for the duration of the treatment of 2 to 4 days and no longer than 6 days depending on the type of cancer.

In certain embodiments of the invention, the systemic concentration of glucose is lowered by infusion of insulin. The goal is to lower plasma glucose concentration to the minimum level needed to supplement ketone bodies delivered by infusion. In healthy individuals on a regular diet, the glucose level is about 5 mM while the ketone level is about 0.5 to 1 mM. The ratio of glucose to ketone (so-called glucose-ketone index, or GKI) is thus about 5 to 10. Infusion of ketone can safely bring its plasma concentration to 4 or 5 mM. Insulin should be used to lower glucose to below about 2 mM, preferably to as low as about 1 mM, inverting the ratio of the two basic sources of energy. Thus, in certain embodiments, GKI in blood is about 1 or less, e.g., about 0.1 to about 0.5.

The insulin may be administered by infusion at a predetermined dose rate, e.g., at a predetermined constant dose rate. For humans, the insulin may be administered at a dose rate from about 0.2 to about 2 IU/kg body weight/day, preferably from about 0.5 to about 1.5 IU/kg body weight//day, more preferably about 1 IU/kg body weight//day. For dogs, the insulin may be administered at a higher dose rate from about 0.4 to about 4 IU/kg body weight//day, preferably from about 1 to about 3 IU/kg body weight//day, more preferably about 2 IU/kg body weight//day.

The insulin may be any type of natural or recombinant insulin or insulin analogue. Preferably, the insulin is a rapid acting insulin or a short acting insulin, more preferably a rapid acting insulin. Examples of rapid acting insulin are insulin lispro, insulin aspart or insulin glulisine. Examples of short acting insulins are regular insulin or insulin velosulin.

Ketone bodies, particularly sodium 3-hydroxybutyrate, have been infused for different pathological conditions but also experimentally in studies of heart performance. Infused at a rate of about 0.2 g/kg/hour, its plasma concentration was increased from zero to between 3 and 4 mM.

In certain embodiments, the therapeutically effective daily dose of an arginase, e.g., ARG1, in a dog is from about 300 to about 6000 IU/kg/day, preferably from about 1000 to about 4500 IU/kg/day, and most preferably 3000 IU/kg/day as determined by experimental work *in vivo.* For humans, a therapeutically effective daily dose is about 150 to about 3000 IU/kg/day, preferably about 500 to about 2000 IU/kg/day, and most preferably about 1500 IU/kg/day. With a specific activity of recombinant ARG1 of about 1000 IU/mg of protein a preferred therapeutically effective daily dose in a dog is about 0.3 to about 6.0 mg/kg/day and in a human about 0.15 to about 3.0 mg/kg/day.

In certain embodiments, the therapeutically effective daily dose of an ASS, e.g., ASS1, in a dog is from about 0.3 to about 6 IU/kg/day, preferably from about 1 to about 4.5 IU/kg/day, and most preferably 3 IU/kg/day. For humans, a therapeutically effective daily dose is about 0.15 to about 3 IU/kg/day, preferably about 0.5 to about 2 IU/kg/day, and most preferably about 1.5 IU/kg/day. With a specific activity of recombinant ASS1 of about 1 IU/mg of protein a preferred therapeutically effective daily dose in a dog is about 0.3 to about 6.0 mg/kg/day and in a human about 0.15 to about 3.0 mg/kg/day.

In certain embodiments, the dose is adapted based on the measurement of arginine and/or citrulline levels in the blood of the subject to be treated. In particular embodiments, the arginine and/or citrulline levels are measured in the plasma of the venous blood.

The medicament is typically administered parenterally, e.g., by injection, or preferably by infusion over a suitable period, for example, over a period from several hours, e.g., at least about 2 hours, or about 6 hours, or even continuously during 1 day up to 6 days, depending on the type of cancer being treated.

Administration of the medicament may be accompanied by certain measures to compensate for side-effects of arginine depletion such as infusion of a nitric oxide (NO) donor, e.g., sodium nitroprusside (SNP), and/or a pressor peptide, e.g., a vasopressin, to balance NO-induced vasodilation. Arginine is the only precursor for the synthesis of short-lived NO. All pressor peptides contain arginine and are short-lived. Co-infusion of Iloprost, a prostacyclin analog has also been found useful in the maintenance of thrombocytes. Infusion of glucose is used only in an emergency should any clinical signs of its lack become manifest.

The medicament is useful for the treatment of cancer including a blood cancer such as leukemia and lymphoma, or a solid cancer particularly selected from liver cancer including primary liver cancer and hepatocellular carcinoma, skin cancer such as melanoma, colon carcinoma, osteosarcoma, soft tissue sarcoma, mast cell tumor, pancreatic cancer, lung cancer, ovarian cancer, prostate cancer, gastric cancer and breast cancer. The medicament is also useful for the treatment of cancer metastases, which are disseminated within the patient's body.

The inventors have found that the efficacy of the medicament may be increased by assisting extravasation of arginase, i.e., the transport from the vascular system into the interstitial fluid. Rapid extravasation of ARG1 in its monomeric form, facilitated by insulin, prevents its elimination by glomerular filtration as described in co-owned application WO 2022/034218 the content of which is herein incorporated by reference.

In certain embodiments, the amino acid-degrading enzyme may be administered to the subject under conditions which promote protein transport into the interstitial fluid. In particular embodiments, the treatment comprises administration of at least amino acid-degrading enzyme under conditions of blood and/or plasma volume expansion. Such a treatment is disclosed in co-owned application EP 23 200 562.9, the content of which is herein incorporated by reference.

The target compartment for the ARG and ASNase is the interstitial volume. This is where most cancer cells reside - only a small fraction of cancer cells are found in the blood at any stage of the disease, even in blood cancers. Enzymatic activity confined to the vascular system cannot efficiently reduce concentrations of amino acids in extravascular volume to very low levels because of the constant influx of amino acids from protein breakdown under homoeostatic mechanisms. The mass transport between intravascular and interstitial fluid (in both directions) is limited by diffusion and modest convection, the rates of which are simply too low when, e.g., arginine is to be systemically reduced into µM range. By contrast, ASS is preferably retained in the blood to intercept the transport of citrulline from the intestines to the kidneys. Its large molecular weight of 186 kDa helps to keep it in the vascular system. Optionally, it could be PEGylated.

It is worth noting that all current clinical trials with arginine depletion as a modality for cancer treatment are conducted with PEGylated enzymes (arginase or arginine deiminase) with high molecular weights that make extravasation extremely limited. This prevents deep systemic arginine depletion needed to kill disseminated cancer. There is also a rapid transition to PEGylated ASNase in some of the markets. This is a very unfortunate, misguided development that makes the problem of ASNase underdosing even worse.

According to the present invention, insulin may be co-administered with ketone bodies, preferably with sodium 3-hydroxybutyrate. Preferably, hydroxybutyrate is administered together with insulin by continuous infusion throughout the treatment period, optionally by oral intake.

## Claims

1. An amino acid-degrading enzyme for use in medicine,
wherein a subject in need thereof is subjected to a treatment with at least one amino acid-degrading enzyme and a blood glucose-lowering agent under conditions of glucose depletion.

2. The amino acid-degrading enzyme for the use of claim 1, wherein the at least one amino acid-degrading enzyme comprises an asparaginase, particularly an asparaginase in a dissociated form, more particularly in the form of monomers.

3. The amino acid-degrading enzyme for the use of claim 2, wherein the at least one amino acid-degrading enzyme comprises an asparaginase preparation comprising a pharmaceutically acceptable chaotropic agent is administered, wherein the chaotropic agent is particularly selected from urea, more particularly urea in a concentration of about 3 mol/l to about 8 mol/l, even more particularly in a concentration of about 4 mol/l to about 6 mol/l, e.g., about 5 mol/l.

4. The amino acid-degrading enzyme for the use of any one of claims 1-3, wherein the treatment with at least one amino acid-degrading enzyme comprises a treatment with
(i) an asparaginase;
(ii) an arginase;
(iii) an argininosuccinate synthase; and
(iv) optionally a glutaminase,
wherein the arginase (ii) and/or the argininosuccinate synthase (iii) are administered externally or provided endogenously.

5. The amino acid-degrading enzyme for the use of claim 4, wherein the asparaginase, the arginase, and the argininosuccinate synthase are administered externally.

6. The amino acid-degrading enzyme for the use of any one of claims 1-5, wherein the blood glucose-lowering agent is an insulin or an insulinotropic peptide, particularly a fast-acting insulin.

7. The amino acid-degrading enzyme for the use of any one of claims 1-6, wherein the treatment further comprises administration of a ketone body compound, e.g., a physiologically acceptable 3-hydroxybutyrate salt.

8. The amino acid-degrading enzyme for the use of any one of claims 1-7, wherein the treatment comprises administration of at least amino acid-degrading enzyme by intravenous infusion.

9. The amino acid-degrading enzyme for the use of any one of claims 1-8, wherein the treatment comprises administration of at least one amino acid-degrading enzyme is administered to the subject under conditions which promote protein transport into the interstitial fluid.

10. The amino acid-degrading enzyme for the use of any one of claims 1-9, wherein the treatment comprises administration of at least amino acid-degrading enzyme under conditions of blood and/or plasma volume expansion.

11. The amino acid-degrading enzyme for the use of any one of claims 1-10, for use in the treatment of cancer.

12. The amino acid-degrading enzyme for the use of claim 11, wherein the cancer is selected from a blood cancer such as leukemia and lymphoma, or a solid cancer such as liver cancer including primary liver cancer, skin cancer such as melanoma, breast cancer, colon cancer, ovarian cancer, lung cancer, prostate cancer, pancreatic cancer, and gastric cancer.

13. The amino acid-degrading enzyme for the use of any one of claims 1-12, wherein the subject is a human.

14. The amino acid-degrading enzyme for the use of any one of claims 1-12, wherein the subject is a non-human mammal, particularly a cat or dog.

15. A method for the treatment of cancer wherein a subject in need thereof is subjected to a treatment with at least one amino acid-degrading enzyme and a blood glucose-lowering agent under conditions of glucose depletion.
